# EUROPEAN PATENT APPLICATION

(11) **EP 4 368 630 A1**
(43) Date of publication of application: **15.05.2024**
(21) Application number: 22837790.9
(22) Date of filing: 28.03.2022
(51) Int. Cl.: C07K 7/06, C07K 16/44, G01N 33/68

(54) **NOVEL ANTIGEN PEPTIDE FOR PREPARATION OF FORMYL-METHIONINE-SPECIFIC ANTIBODY**

(30) Priority: 09.07.2021 KR 20210090288
(71) Applicant: POSTECH Research and Business Development Foundation, Pohang-si, Gyeongsangbuk-do 37673 (KR)
(72) Inventor: HWANG, Cheol Sang, Gyeongsangbuk-do 37673 (KR); KIM, Da Som, Pohang-si, Gyeongsangbuk-do 37673 (KR); KIM, Jeong Mok, Seoul 37887 (KR); SEOK, Ok Hee, Gyeongsangbuk-do 37673 (KR)
(74) Representative: Cabinet Becker et Associés
(86) International application number: PCT/KR2022/004347
(87) International publication number: WO 2023/282436

(57) **Abstract**

The present invention relates to a novel antigen peptide and, more specifically, the antigen peptide is for preparing an antibody capable of specifically detecting formyl-methionine. It has been identified that an antibody prepared using an antigen peptide for the preparation of a formyl-methionine-specific antibody, according to the present invention, has excellent antigen-binding ability at peptide and protein levels and has excellent antigen-binding ability even under a condition in which intracellular formylated protein expression changes. Therefore, an antigen peptide of the present invention can be effectively used in the preparation of antibodies universally recognizing peptides and proteins having formyl-methionine.

## Description

### [Technical Field]

The present invention relates to a novel antigen peptide and, more particularly, the antigen peptide is for preparing an antibody capable of specifically detecting formyl-methionine.

### [Background Art]

An antibody is a substance that specifically binds to an antigen to cause an antigen-antibody reaction, and this reaction serves to remove foreign antigens such as bacteria and viruses from the human body. The antibody has been used to detect a specific antigen in various experiments within life science research, and representative experiments include western blot assay, immunoprecipitation, immunohistochemistry, and the like. For this purpose, numerous antibodies are commercially available, and target antigens are mainly specific proteins or protein modifications.

N-terminal formylation is a type of protein modification, and is known to be mainly observed in bacteria, mitochondria, and chloroplasts, and observed even in the cytoplasm of eukaryotic cells under specific stress situations. An enzyme that plays a major role in the formylation of intracellular proteins is formylmethionyl-transferase, and this enzyme attaches a formyl group to methionyl-tRNA to create formylmethionyl-tRNA. Thereafter, when the formylmethionyl-tRNA is used to initiate protein synthesis, proteins containing formyl-methionine at the N-terminal are synthesized.

Methods for preparing antibodies to specifically detect N-terminal formylation of proteins have been proposed for a long time. For example, to prepare an antibody that recognizes a peptide called formyl-MLF, a method of preparing an antibody by attaching the formyl-MLF peptide to a carrier protein to be used as an antigen has been used. However, this method has a disadvantage of not recognizing other formyl peptides or proteins having different sequences from the target formyl peptide. Therefore, when using existing methods, antibodies recognizing each formyl peptide need to be prepared separately, and when the diversity of protein sequences is considered, inefficiency becomes noticeable.

Accordingly, there is a need for a method for overcoming the limitations of the prior art and for producing and purifying an antibody capable of universally recognizing formyl-methionine.

### [Disclosure]

### [Technical Problem]

The present inventors developed a novel antigen peptide capable of preparing an antibody capable of universally recognizing formyl-methionine, confirmed that the antibody prepared using the antigen peptide had excellent antigen-binding ability to formyl-methionine at peptide and protein levels, and then completed the present invention.

Accordingly, an object of the present invention is to provide an antigen peptide comprising the amino acid sequence shown in SEQ ID NO: 1, in which a first methionine is formylated.

Another object of the present invention is to provide a composition for preparing a formyl-methionine-specific antibody including an antigen peptide comprising the amino acid sequence shown in SEQ ID NO: 1, in which a first methionine is formylated.

Yet another object of the present invention is to provide a kit for preparing a formyl-methionine-specific antibody including an antigen peptide comprising the amino acid sequence shown in SEQ ID NO: 1, in which a first methionine is formylated.

Yet another object of the present invention is to provide a method for preparing a formyl-methionine-specific antibody including (a) inducing an immune response by administering the antigen peptide according to claim 1 to a host; (b) isolating serum from the host in which the immune response of step (a) is induced; and (c) purifying a formyl-methionine-specific antibody from the serum of step (b), and a formyl-methionine-specific antibody prepared by the method.

Yet another object of the present invention is to provide a method for detecting a peptide or protein having formyl-methionine as a first amino acid, including inducing an antigen-antibody reaction by treating a sample with a formyl-methionine-specific antibody prepared by the method for preparing the formyl-methionine-specific antibody.

### [Technical Solution]

In order to achieve the object, an aspect of the present invention provides an antigen peptide comprising the amino acid sequence shown in SEQ ID NO: 1, in which a first methionine is formylated.

In addition, another aspect of the present invention provides a composition for preparing a formyl-methionine-specific antibody including an antigen peptide comprising the amino acid sequence shown in SEQ ID NO: 1, in which a first methionine is formylated.

In addition, another aspect of the present invention provides a kit for preparing a formyl-methionine-specific antibody including an antigen peptide comprising the amino acid sequence shown in SEQ ID NO: 1, in which a first methionine is formylated.

In addition, yet another aspect of the present invention provides a method for preparing a formyl-methionine-specific antibody including (a) inducing an immune response by administering the antigen peptide to a host; (b) isolating serum from the host in which the immune response of step (a) is induced; and (c) purifying a formyl-methionine-specific antibody from the serum of step (b), and a formyl-methionine-specific antibody prepared by the method.

In addition, yet another aspect of the present invention provides a method for detecting a peptide or protein having formyl-methionine as a first amino acid, including inducing an antigen-antibody reaction by treating a sample with a formyl-methionine-specific antibody prepared by the method for preparing the formyl-methionine-specific antibody.

### [Advantageous Effects]

According to the present invention, it has been identified that an antibody prepared using an antigen peptide for the preparation of a formyl-methionine-specific antibody has excellent antigen-binding ability at peptide and protein levels and has excellent antigen-binding ability even under a condition in which intracellular formylated protein expression changes. Therefore, an antigen peptide of the present invention can be effectively used in the preparation of antibodies universally recognizing peptides and proteins having formyl-methionine.

### [Description of Drawings]

FIG. 1 is a diagram illustrating a method for preparing an antibody using an antigen for preparing a formyl-methionine-specific antibody according to the present invention.
FIG. 2 is a diagram illustrating results of confirming antigen-binding ability of the formyl-methionine-specific antibody of the present invention at a peptide level through dot blot.
FIG. 3 is a diagram illustrating results of confirming antigen-binding ability of the formyl-methionine-specific antibody of the present invention at a protein level through Western blot.
FIG. 4 is a diagram illustrating results of confirming antigen-binding ability of the formyl-methionine-specific antibody of the present invention according to the expression of E. coli-derived formylation-related enzymes in HeLa cells through Western blot.
FIG. 5 is a diagram illustrating results of confirming antigen-binding ability of the formyl-methionine-specific antibody of the present invention in cancer cells through Western blot.

### [Best mode of the Invention]

Hereinafter, the present invention will be described in detail.

According to an aspect of the present invention, the present invention provides an antigen peptide comprising the amino acid sequence shown in SEQ ID NO: 1, in which a first methionine is formylated.

In the present invention, the peptide refers to a linear molecule formed by linking amino acid residues with each other by peptide bonds. The peptide may be prepared according to a chemical synthesis method known in the art, preferably prepared according to a solid-phase synthesis technique, but is not limited thereto.

In addition, the range of the antigen peptide of the present invention includes functional equivalents of the peptide containing the amino acid sequence represented by SEQ ID NO: 1, more preferably functional equivalents of the peptide containing the amino acid sequence represented by SEQ ID NO: 1, and salts thereof.

The functional equivalents refer to peptides which have sequence homology (that is, identity) of at least 75% or more, preferably 90%, more preferably 95% or more with the peptide represented by SEQ ID NO: 1, for example, sequence homology of 80%, 81%, 82%, 83%, 84%, 85%, 86%, 87%, 88%, 89%, 90%, 91%, 92%, 93%, 94%, 95%, 96%, 97%, 98%, 99%, and 100%, as a result of addition, substitution or deletion of amino acids, and exhibit substantially homogeneous physiological activity as the peptide consisting of the amino acid sequence shown in SEQ ID NO: 1. In the present specification, the sequence homology and homogeneity are defined as a percentage of amino acid residues of a candidate sequence to the amino acid sequence represented by SEQ ID NO: 1 after aligning the amino acid sequence represented by SEQ ID NO: 1 and the candidate sequence and introducing gaps. If necessary, conservative substitution is not considered as part of sequence homogeneity in order to obtain the maximum percentage sequence homogeneity. An N-terminal, a C-terminal or internal extension, deletion or insertion of the amino acid sequence represented by SEQ ID NO: 1 is not construed as a sequence affecting sequence homogeneity or homology.

In addition, the sequence homogeneity may be determined by a general standard method used to compare similar portions of amino acid sequences of two polypeptides. A computer program such as BLAST or FASTA aligns the two polypeptides so as to optimally match respective amino acids (according to a fulllength sequence of one or two sequences, or a predicted portion of one or two sequences). The program provides a default opening penalty and a default gap penalty and provides a scoring matrix such as PAM250 (standard scoring matrix) which may be used in association with the computer program. For example, the percentage homogeneity may be calculated as follows. The total number of identical matches is multiplied by 100 and then divided into a sum of the length of a longer sequence in a corresponding matched span and the number of gaps introduced into the longer sequence to align the two sequences.

The range of the functional equivalents of the present invention includes derivatives in which some chemical structures of the peptide are modified while maintaining the basic skeleton of the peptide represented by SEQ ID NO: 1. For example, the range of the functional equivalents includes structural modifications for changing the stability, storage, volatility, solubility or the like of the peptide.

In the present invention, the formylation refers to the action of introducing a formyl group (CHO-) into a compound. In the present invention, the `formylation' refers to formylation of a α-amino group of an amino acid.

In an embodiment of the present invention, a fifth amino acid 'X' of the antigen peptide is used to attach the antigen peptide to the carrier protein, and preferably cysteine.

In an embodiment of the present invention, the antigen further includes preferably a carrier protein, more preferably at least one carrier protein selected from the group consisting of Keyhole Limpet Hemocyanin (KLH), bovine serum albumin (BSA), and ovalbumin (OVA), but is not limited thereto.

In a preferred embodiment of the present invention, the antigen peptide is preferably operably linked in the form of Structural Formula 1 below.

### [Structural Formula 1]

### Antigen peptide (SEQ ID NO: 1) - Carrier protein

In the present invention, `operably linked' means that amino acids, peptides, or proteins are linked in an appropriate manner (for example, linkage of a Cys group of the antigen peptide and a Lys group of the carrier protein KLH using Sulfo-SMCC) to exhibit specific activity.

In an embodiment of the present invention, the antigen peptide is preferably used for preparing an antibody specific to formyl-methionine.

`GSG', the second and fourth amino acids of the antigen peptide according to the present invention, has a short side chain, and when preparing an antibody using the short side chain, a structure of the antibody is formed by focusing on formyl methionine, the first amino acid of the antigen peptide. Accordingly, the formyl-methionine-specific antibody prepared with the antigen peptide of the present invention may bind more universally to a peptide or protein having formyl-methionine as the first amino acid.

According to another aspect of the present invention, the present invention provides a composition for preparing a formyl-methionine-specific antibody including an antigen peptide comprising the amino acid sequence shown in SEQ ID NO: 1, in which a first methionine is formylated. In addition, the present invention provides a kit for preparing a formyl-methionine-specific antibody including an antigen peptide comprising the amino acid sequence shown in SEQ ID NO: 1, in which a first methionine is formylated.

In an embodiment of the present invention, the antibody preferably detects the first formyl-methionine regardless of a second amino acid type of the peptide or protein.

The composition and kit for preparing the antibody according to the present invention may further include known ingredients for maintaining and preserving the antigen peptide.

In an embodiment of the present invention, the composition and kit for preparing the antibody may further include known agents for inducing and promoting an immune response in the host.

The kit for preparing the formyl-methionine-specific antibody according to the present invention may include the composition for preparing the formyl-methionine-specific antibody of the present invention.

According to yet another aspect of the present invention, the present invention provides a method for preparing a formyl-methionine-specific antibody including (a) inducing an immune response by administering the antigen peptide according to claim 1 to a host; (b) isolating serum from the host in which the immune response of step (a) is induced; and (c) purifying a formyl-methionine-specific antibody from the serum of step (b). In addition, the present invention provides a formyl-methionine-specific antibody prepared by the method.

In an embodiment of the present invention, the host is preferably at least one selected from the group consisting of mouse, rabbit, horse, dog, cat, chicken, and duck, more preferably rabbit, but is not limited thereto.

The method for preparing the formyl-methionine-specific antibody of the present invention may further include boosting antibody formation before step (b).

In an embodiment of the present invention, the antibody may be a monoclonal antibody or a polyclonal antibody.

In addition, the antibody according to the present invention has a technical feature of being versatile, and thus has an advantage of being able to specifically detect formyl-methionine in mammalian cells, yeast, *E. coli,* cancer cells, etc.

According to yet another aspect of the present invention, the present invention provides a method for detecting a peptide or protein having formyl-methionine as a first amino acid, including inducing an antigen-antibody reaction by treating a sample with a formyl-methionine-specific antibody prepared by the method for preparing the formyl-methionine-specific antibody.

Duplicated contents are omitted in consideration of the complexity of the present specification, and terms not defined otherwise in the present specification have the meanings commonly used in the art to which the present invention pertains.

### [Modes for the Invention]

Hereinafter, the present invention will be described in more detail through Examples. These Examples are just illustrative of the present invention, and it will be apparent to those skilled in the art that it is not interpreted that the scope of the present invention is limited to these Examples.

### Example 1. Preparation of formyl-methionine-specific antibody

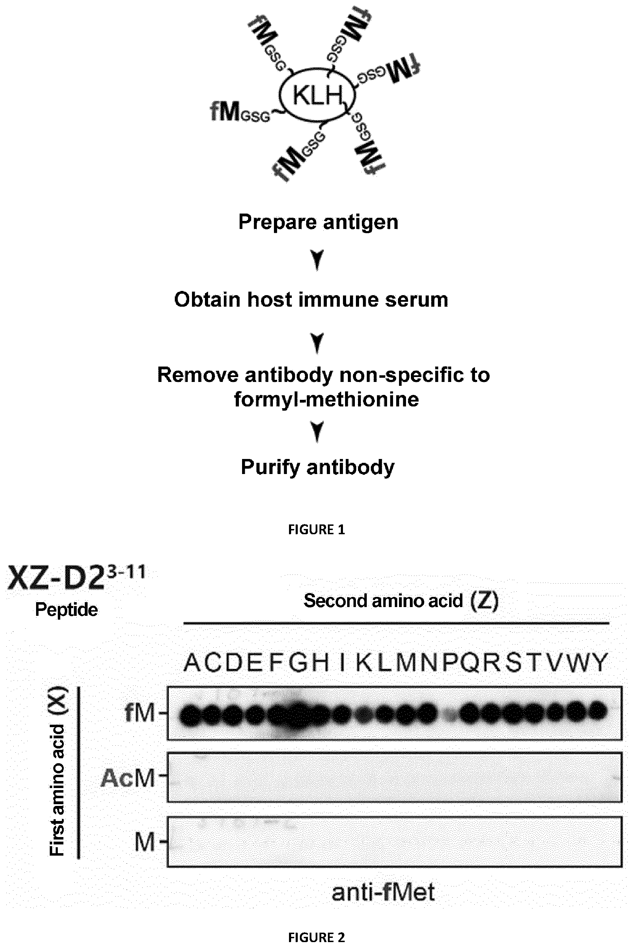

A method for preparing a formyl-methionine-specific antibody was illustrated in FIG. 1.

### 1-1. Preparation of antigen

A peptide formyl-MGSG-C, which had a formyl group at an N-terminal, was synthesized. An antigen was prepared by attaching the synthesized peptide formyl-MGSG-X (SEQ ID NO: 1) to a carrier protein 'Keyhole Limpet Hemocyanin (KLH)'.

In Examples to be described below, there was used an antigen prepared by attaching a peptide (that is, formyl-MGSG-C) having the fifth amino acid `X' represented by SEQ ID NO: 1, 'cysteine (C)' to the carrier protein 'Keyhole Limpet Hemocyanin.'

### 1-2. Preparation and purification of antibody

First, proteins to be filled into a column for antibody purification were prepared. A pcDNA3.1-EcPDF (E. coli-derived peptide deformylase) vector was delivered and expressed in a HeLa cell line through PEI to create conditions in which a formyl protein was reduced. Specifically, the HeLa cell line was cultured in a 100 cm dish and a DMEM + 10 % FBS 10 ml medium. The next day, when the growth reached 70 to 80%, 7 µg of the vector was mixed with 28 µl PEI material and 1 ml OPTI-MEM, stored for 30 minutes, and then treated with the cultured cells. After 24 hours, cells were harvested using a scraper. 70 dishes of HeLa cells obtained under the conditions were lysed with a 30 ml lysis buffer (50 mM Tris-HCl pH 7.6, 150 mM NaCl, 1% NP-40, 1 mM DTT, 1x protease inhibitor cocktail), and the buffer was exchanged with a conjugation buffer (20 mM HEPES pH 7.6, 100 mM NaCl, 0.5 mM EDTA, 10% glycerol, 0.1 mM PMSF, 1 mM DTT). Through this process, a large amount of protein mixture was obtained, and the protein mixture reacted with 2 ml of a resin mixed with Affi-gel 10/15 in a 1 : 1 ratio to secure a resin attached with the protein mixture.

The antigen prepared in Example 1-1 was injected into rabbits to induce an immune response. After the boosting process in weeks 4, 6, and 8, about 60 ml of serum was obtained in week 9. In order to remove antibodies non-specific to formylmethionine, 3 ml of the obtained rabbit serum reacted with the resin attached with the protein mixture at 4°C for 4 hours or more. The process of removing the non-specific antibodies through the resin attached with the protein mixture was repeated a total of six times. Thereafter, only the formyl-methionine-specific antibody was isolated using 500 µl of ProA-agarose resin.

### Example 2. Identification of antigen-binding ability of formyl-methionine-specific antibody at peptide level

A modified peptide was prepared by modifying the first and second amino acids of a peptide (MTIAIGTYQEK) derived from an N-terminal of a D2 protein. The modified peptide had the first amino acid of formyl-methionine (fM), acetylmethionine (AcM), or methionine (M), and the second amino acid of 20 other amino acids (A, C, D, E, F, G, H, I, K, L, M, N, P, Q, R, S, T, V, W, and Y). The binding of each peptide to the formyl-methionine-specific antibody was analyzed by dot blot.

Specifically, a membrane custom-made by JPT was soaked with a small amount of methanol for 5 minutes. The membrane was washed with 5 ml of TBS-T (50 mM TRIS, 137 mM NaCl, 2.7 mM KCl, adjust pH to 8.0 with HCl, and then 0.05 % Tween 20 supplemented) for 3 minutes (room temperature/repeated 3 times). The membrane was added with 5 ml of a blocking buffer (Super block T20, ThermoFisher #37536) and soaked on a rocker for one day (4°C). The formyl-methionine-specific antibody was added to 5 ml of the blocking buffer at a concentration of 0.8 µg/ml and soaked on a rocker for one day (4°C). The membrane was washed with 5 ml of TBS-T using a shaker for 5 minutes (room temperature/repeated 3 times). An anti-Rabbit HRP secondary antibody (Bio-Rad, #1706515) was added to 5 ml of the blocking buffer in a ratio of 1 : 5000 and the membrane was soaked on the rocker for 2 hours (room temperature). The membrane was washed with 5 ml of TBS-T using a shaker for 5 minutes (room temperature/repeated 3 times). The membrane was evenly wet with 1 ml of an ECL substrate (Clarity Western ECL Substrate, Bio-Rad #1705061) and the results were obtained using AI680 chemiluminescence. The dot blot results were illustrated in FIG. 2.

As illustrated in FIG. 2, it has been identified that the formyl-methionine-specific antibody prepared in Example 1 was bound only when the first amino acid of the peptide was formyl-methionine, regardless of the second amino acid.

### Example 3. Identification of antigen-binding ability of formyl-methionine-specific antibody at protein level

Samples were prepared by mixing 200 ng of purified proteins MD-D2(3-11)-GST, fMD-D2(3-11)-GST, Pgd1-HA-His6, and fPgd1-HA-His6 with a 4X SDS sample buffer (200 mM Tris-HCl pH 6.8, 8% SDS, 40% glycerol, 4% β-mercaptoethanol, 50 mM EDTA, 0.08 % bromophenol blue) to be 1X. Western blot was performed using the prepared samples. Specifically, first, a membrane was obtained using the samples by SDS-PAGE electrophoresis and transfer.

### <Western blot using membrane for Anti-fMet>

The membrane was added with 5 ml of a blocking buffer (5 % skim milk in PBS-T) and soaked on a rocker for 1 hour (room temperature). The membrane was washed with 5 ml of PBS-T for 5 minutes using a shaker. The formyl-methionine-specific antibody was added with 5 ml of PBS-T (NaCl 137 mM, KCl 2.7 mM, Na₂HPO₄ 10 mM, KH₂PO₄ 1.8 mM, Tween-20 0.1% (w/v)) at a concentration of 2 µg/ml and then the membrane was soaked on the rocker for one day (4°C). The membrane was washed with 5 ml of PBS-T using a shaker for 5 minutes (room temperature/repeated 3 times). An anti-Rabbit HRP secondary antibody (Bio-Rad, #1706515) was added to 5 ml of PBS-T in a ratio of 1 : 5000 and the membrane was soaked on the rocker for 1 hour (room temperature). After the treatment of the secondary antibody, the membrane was washed with 5 ml of PBS-T using a shaker for 5 minutes (room temperature/repeated 3 times). The membrane was evenly wet with 1 ml of an ECL substrate (Clarity Western ECL Substrate, Bio-Rad #1705061) and the results were obtained using AI680 chemiluminescence.

### <Western blot using membrane for Anti-GST, ha, flag, and α-tubulin>

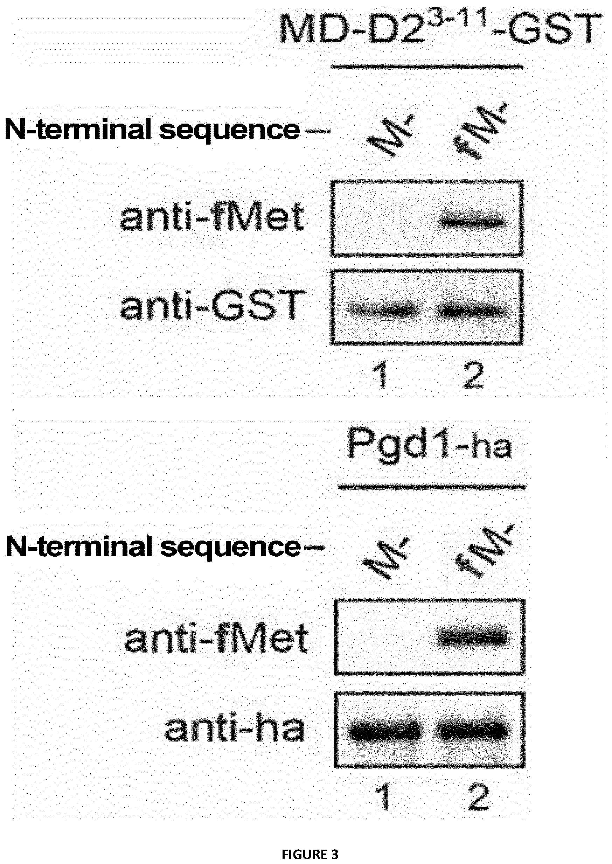

The membrane was added with 5 ml of a blocking buffer and soaked on a rocker for 1 hour (room temperature). The membrane was added with 5 ml of a blocking buffer containing a GST, ha, flag, and α-tubulin specific antibodies (Santacruz #374171, Sigma #H9658, Sigma #F3165, Sigma #T5168) in a 1 : 2000 ratio and soaked on the rocker for one day (4°C). The membrane was washed with 5 ml of PBS-T using a shaker for 5 minutes (room temperature/repeated 3 times). An antimouse HRP secondary antibody (Bio-Rad, #1706516) was added with 5 ml of the blocking buffer in a ratio of 1 : 10000 and the membrane was soaked on the rocker for 1 hour (room temperature). The membrane was washed with 5 ml of PBS-T using a shaker for 5 minutes (room temperature/repeated 3 times). The membrane was evenly wet with 1 ml of a ECL substrate and the results were obtained using AI680 chemiluminescence. The Western blot results were illustrated in FIG. 3.

As illustrated in FIG. 3, it has been identified that the formyl-methionine-specific antibody was bound specifically to a protein (Lane 2) containing formylmethionine compared to a protein (Lane 1) containing unformylated methionine.

### Example 4. Identification of antigen-binding ability of formyl-methionine-specific antibody According to expression of E. coli-derived formylation-related enzymes in HeLa cells

The antigen-binding ability of a formyl-methionine-specific antibody was identified according to the expression of formyl-methionine transferase and deformylase in HeLa cells. Specifically, first, each combination of vectors was expressed in the HeLa cells using Lipofectamine 2000 and harvested, and then the proteins were extracted and mixed with a 4X SDS sample buffer to be 1X to prepare samples and then the samples were loaded.

The ingredients of the prepared sample were as follows.
Lane 1: pcDNA3.1-formylmethionine-transferase-3flag + pcDNA3.1 empty vector
Lane 2: pcDNA3.1-formylmethionine-transferase-3flag + pcDNA3.1-deformylase-3flag
Lane 3: pcDNA3.1-formylmethionine-transferase-3flag + pcDNA3.1-deformylase mutant (E134A)-3flag

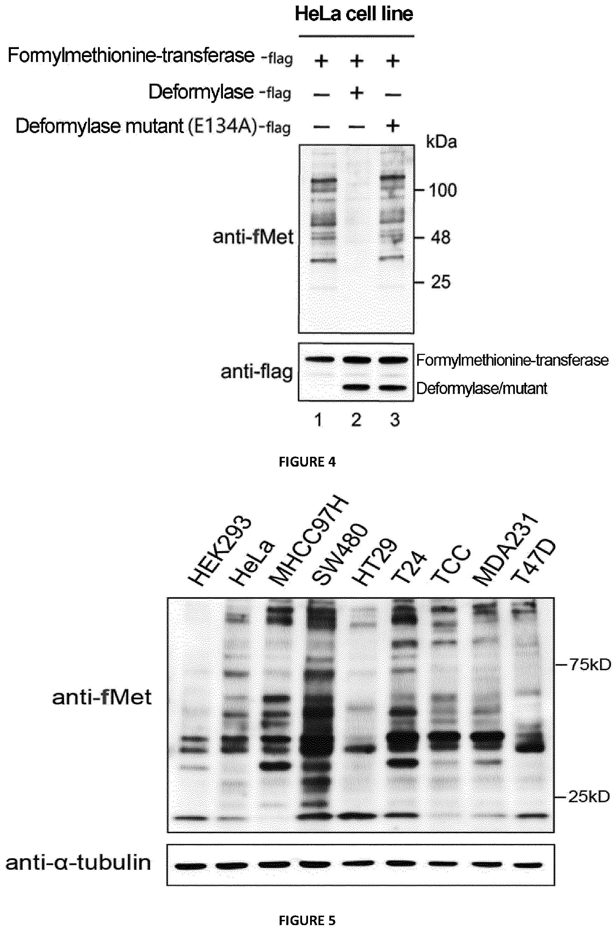

The gel loaded with the samples was subjected to electrophoresis, and the proteins were transferred to the membrane. Western blot was performed using a membrane containing the proteins, and the results were illustrated in FIG. 4.

As illustrated in FIG. 4, it has been identified that even in conditions of an increase (expression of *E. coli*-derived formylmethionine-transferase); or decrease (expression of *E. coli*-derived formylmethionine-transferase + expression of deformylase) of intracellular formylated protein, the formyl-methionine-specific antibody was specifically bound to the antigen containing formyl-methionine.

### Example 5. Identification of antigen-binding ability of formyl-methionine-specific antibody in cancer cells

The antigen-binding ability of the formyl-methionine-specific antibody has been identified in non-cancer cells (HEK293) and various cancer cells (HeLa, MHCC97H, SW480, HT29, T24, TCC, MDA231, and T47D). Specifically, each cell line was cultured in DMEM (supplemented with 10% FBS) or RPMI (supplemented with 10% FBS), and then cell pellets were obtained using trypsin. The cell pellets were dissolved in RIPA buffer (supplemented with 1x protease inhibitor + 20 µg/ml actinonin), incubated on ice for 20 minutes, sonicated, and the cell lysates were obtained through centrifugation (12000 rpm, 4°C for 10 minutes). The concentration of the lysate corresponding to each cell line was measured using a Bradford assay, and the lysate was mixed with a SDS sample buffer and boiled at 70°C for 10 minutes to prepare samples for Western blot. Western blot was performed after loading the samples on Bio-Rad 4-20% Mini-PROTEAN^{®}TGX^{™} Precast Protein Gel. The results of the obtained membranes were confirmed by the method specified in Example 3. The Western blot results were illustrated in FIG. 5.

As illustrated in FIG. 5, it has been identified that the formyl-methionine-specific antibody prepared in Example 1 could be specifically detected formylated proteins in various cancer cells (HeLa, MHCC97H, SW480, HT29, T24, TCC, MDA231, T47D).

Overall, the present inventors prepared a novel antigen (formyl-MGSG-C) for preparing a formyl-methionine-specific antibody and prepared a polyclonal antibody using the same. It has been identified that the prepared formyl-methionine-specific antibody had excellent antigen-binding ability at peptide and protein levels and had significantly high antigen-binding ability even under a condition in which intracellular formylated protein expression changed. The antigen (formyl-MGSG-C) of the present invention may be effectively used in the preparation of antibodies universally recognizing peptides and proteins having formyl-methionine.

As described above, specific parts of the present invention have been described in detail, and it will be apparent to those skilled in the art that these specific techniques are merely preferred embodiments, and the scope of the present invention is not limited thereto. Therefore, the substantial scope of the present invention will be defined by the appended claims and their equivalents.

## Claims

1. An antigen peptide comprising the amino acid sequence shown in SEQ ID NO: 1, wherein the first methionine of the antigen peptide is formylated.

2. The antigen peptide of claim 1, wherein the fifth amino acid 'X' of the antigen peptide is cysteine.

3. The antigen peptide of claim 1, further comprising:
a carrier protein.

4. The antigen peptide of claim 3, wherein the antigen peptide is operably linked in the form of Structural Formula 1 below.
[Structural Formula 1]
Antigen peptide (SEQ ID NO: 1) - Carrier protein

5. The antigen peptide of claim 1, wherein the antigen peptide is used for preparing an antibody specific to formyl-methionine.

6. A composition for preparing a formyl-methionine-specific antibody comprising an antigen peptide comprising the amino acid sequence shown in SEQ ID NO: 1, wherein the first methionine of the antigen peptide is formylated.

7. The composition for preparing the formyl-methionine-specific antibody of claim 6, wherein the antibody detects the first formyl-methionine regardless of the second amino acid type of the peptide or protein.

8. A kit for preparing a formyl-methionine-specific antibody comprising an antigen peptide comprising the amino acid sequence shown in SEQ ID NO: 1, wherein the first methionine of the antigen peptide is formylated.

9. A method for preparing a formyl-methionine-specific antibody comprising:
(a) inducing an immune response by administering the antigen peptide according to claim 1 to a host;
(b) isolating serum from the host in which the immune response of step (a) is induced; and
(c) purifying a formyl-methionine-specific antibody from the serum of step (b).

10. The method for preparing the formyl-methionine-specific antibody of claim 9, wherein the host is at least one selected from the group consisting of mouse, rabbit, horse, dog, cat, chicken, and duck.

11. A formyl-methionine-specific antibody prepared by the method of claim 9.

12. A method for detecting a peptide or protein having formyl-methionine as the first amino acid, comprising inducing an antigen-antibody reaction by treating a sample with the formyl-methionine-specific antibody prepared by the method of claim 9.
